# EUROPEAN PATENT APPLICATION

(11) **EP 4 357 339 A1**
(43) Date of publication of application: **24.04.2024**
(21) Application number: 22827172.2
(22) Date of filing: 24.04.2022
(51) Int. Cl.: C07D 401/12, C07D 401/14, C07D 409/14, C07D 413/14, C07D 487/04, C07D 417/14, A61P 37/02, A61P 29/00, A61P 11/00, A61P 1/00, A61P 37/08, A61P 31/00, A61P 17/02, A61P 27/02, A61P 19/02, A61P 19/08, A61P 21/00, A61P 17/00, A61P 13/12, A61P 7/00

(54) **SULFOXIMIDE SUBSTITUTED INDAZOLE IRAK4 KINASE INHIBITOR, PREPARATION METHOD THEREFOR, AND USE THEREOF**

(30) Priority: 21.06.2021 CN 202110684539
(71) Applicant: Shanghai Xunhe Pharmaceutical Technology Co. Ltd., Shanghai 200000 (CN)
(72) Inventor: WEI, Nongnong, Shanghai 200000 (CN); JIN, Hua, Shanghai 200000 (CN); ZHENG, Yongyong, Shanghai 200000 (CN); ZHOU, Feng, Shanghai 200000 (CN); HUANG, Meihua, Shanghai 200000 (CN)
(74) Representative: Sun, Yanan
(86) International application number: PCT/CN2022/088752
(87) International publication number: WO 2022/267673

(57) **Abstract**

The present invention relates to the technical field of biomedicine, in particular to a sulfoximide substituted indazole compound, an isomer thereof, or a pharmaceutically acceptable salt thereof. The structure of the sulfoximide substituted indazole compound is shown by formula I:

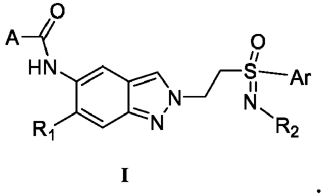

## Description

### TECHNICAL FIELD

The present invention relates to the technical field of biomedicine, in particular to an aromatic (hetero) sulfoximide substituted indazole IRAK4 kinase inhibitor, an isomer thereof, or a pharmaceutically acceptable salt thereof, preparation method thereof and use thereof.

### BACKGROUND

Interleukin-1 receptor associated kinase 4 (IRAK-4) is a member of the intracellular serine-threonine kinase IRAK family. Other members of the kinase family also include IRAK-1, IRAK-2, and IRAK-M. IRAK-M is only expressed in monocytes and macrophages, while the expression of IRAK-1, IRAK-2, and IRAK4 is ubiquitous. IRAK4 is mainly composed of N-terminal conserved death domain (DD), hinge region, and C-terminal central kinase domain (KD). The death domain (DD) is the domain where IRAK4 binds to adaptor protein myeloid differentiation factor primary response gene 88 (MyD88). The kinase domain (KD) consists of 12 subdomains and exhibits typical serine-threonine kinase domain characteristics. The main function of IRAK4 is to phosphorylate its substrate through the kinase domain, thereby activating downstream signaling molecules. IRAK4 is a key factor downstream of the interleukin-1 receptor (IL-1R)/Toll-like receptor (TLR)-mediated inflammatory signal transduction pathway and plays a key role in the immune system. (Sims JE, et al. Nat Rev Immunol, 2010,10 (2): 89-102). When the interleukin-1 receptor (IL-1R) or Toll-like receptor (TLR) binds to the ligand, IRAK4 can mediate signal transduction and activate the expression of downstream inflammatory factors. TLR can receive ligand signals generated by the interaction between body and microorganism or endogenous substance stimulation, as well as the first wave of inflammatory signals and innate immune response signals triggered by these stimuli. TLR plays a crucial role in many diseases, including infection and autoinflammatory disease as well as many other human diseases. Like tumor necrosis factor-a (TNF-α) and other major cytokines, interleukin-1 (IL-1) is a key factor in the inflammation-mediated pathway that is able to transmit and amplify signals. Due to the cross-linking effect of TLR, IL-1R, and other cytokine receptor mediated signaling pathways, IRAK4, a key signaling factor in the TLR and IL-1R inflammatory pathways, plays a significant role in systemic inflammatory response and can serve as an effective potential target for the treatment of various inflammation related diseases.

Testing revealed that a proportion of human patients lack IRAK4 expression (Picard, C. Et al., 2003, Science 299: 2076-2079), and cells obtained from these patients did not respond to all TLR (except TLR3) agonists and IL-1 family members (including IL-1β and IL-18) (Ku, C. Et al, 2007, J. Exp. Med. 204: 2407-2422). Deletion of IRAK4 in mice resulted in severe blockade of IL-1, IL-18, and all TLR (except TLR3)-dependent responses (Suzuki, N. Et al., 2002, Nature 416: 750-754). In contrast, deletion of IRAK1 (Thomas, J. A. Et al., 1999, J. Immunol. 163: 978-984; Swantek, J. L. Et al., 2000, J. Immunol. 164: 4301-4306) or IRAK2 (Wan, Y. Et al., 2009, J. Biol. Chem. 284: 10367-10375) results in obstruction of signal transduction part only. Moreover, IRAK4 is the only family member of the IRAK family whose kinase activity has been shown to be essential for initiating signal transduction. Substitution of wild-type IRAK4 in mouse genome with a kinase-inactive mutant (KDKI) blocks all signals transmitted by MyD88-dependent receptors, including IL-1, IL-18, and all TLRs (except TLR3) (Koziczak-Holbro, M. et al., 2007, J. Biol. Chem. 282: 13552-13560; Kawagoe, T. et al., 2007, J. Exp. Med. 204: 1013-1024).

Compared with wild-type mice, mice with a kinase-inactive mutant (KDKI) showed a significant reduction in disease severity on disease models of multiple sclerosis (Staschke, K.A. et al., 2009, J.Immunol.183: 568-577), rheumatoid arthritis (Koziczak-Holbro, M. et al., 2009, Arthritis Rheum. 60: 1661-1671), atherosclerosis (Kim, T. W. et al., 2011, J. Immunol. 186: 2871-2880) and myocardial infarction (Maekawa, Y. Et al., 2009, Circulation120: 1401-1414). As mentioned above, IRAK4 inhibitors can block all MyD88-dependent signal transduction. MyD88-dependent TLRs have been shown to be the cause of the following diseases: multiple sclerosis, rheumatoid arthritis, cardiovascular disease, metabolic syndrome, pyaemia, systemic lupus erythematosus, inflammatory bowel disease (including Crohn's disease and ulcerative colitis), autoimmune uveitis, asthma, allergy, type I diabetes, and rejection after organ transplantation (Keogh, B. Et al., 2011, Trends Pharmacol. Sci. 32: 435-442; Mann, D. L. 2011, Circ. Res. 108: 1133-1145; Goldstein, D. R. Et al., 2005, J. Heart Lung Transpl. 24: 1721-1729; and Cario, E., 2010, Inflamm. Bowel Dis. 16: 1583-1597). In diffuse large B-cell lymphoma, tumor cells with oncogenic MyD88 mutations have been identified to be sensitive to IRAK4 inhibition (Ngo, V. Et al., 2011, Nature 470: 115-121). Whole-genome sequencing has also confirmed that MyD88 mutations are associated with chronic lymphocytic leukemia, indicating the possibility of IRAK4 inhibitors being used to treat leukemia (Puente, X. S. Et al., 2011, Nature 475: 101-105).

IRAK4 inhibitors can also block signals transmitted by IL-1 and the IL-1 family. Regulation of IL-1 has been proved to be effective for a variety of diseases, including gout, gouty arthritis, type II diabetes, autoinflammatory disease, tumor necrosis factor receptor-associated periodic syndrome, familial Mediterranean fever, adult Still disease, systemic-onset juvenile idiopathic arthritis, stroke, graft-versus-host disease, asymptomatic multiple myeloma, recurrent pericarditis, osteoarthritis and emphysema, etc. (Dinarello, C. A., 2011, Eur. J. Immunol. 41; 1 203-1217; and Couillin, I. et al., 2009. J. Immunol. 183: 8195-8202). In the mouse model of Alzheimer's disease, blocking the IL-1 receptor improved cognitive deficits, reduced Tau protein lesions, and reduced the oligomeric form of amyloid-β (Kitazawa, M. et al., 2011. J. Immunol. 187: 6539-6549). IL-1 has also been proven to be a key link in acquired immunity, driving the differentiation of effector T cell subset Th17 (Chung, Y. et al., 2009, Immunity 30: 576-587). Therefore, IRAK4 inhibitors are predicted to show efficacy in Th17 cell-related diseases, including multiple sclerosis, psoriasis, inflammatory bowel disease, autoimmune uveitis, and rheumatoid arthritis, etc. (Wilke, C. M. Et al., 2011, Trends Immunol. 32: 603-611).

Pulmonary diseases such as pulmonary fibrosis, chronic obstructive pulmonary disease (COPD), acute respiratory distress syndrome (ARDS), acute lung injury (ALI), interstitial lung disease (ILD), sarcoidosis, and pulmonary hypertension have also been shown to be associated with various TLR-mediated signaling pathways. The pathogenesis of lung disorders may be either infection-mediated process or non-infection-mediated process (Jeyaseelan, Chu et al., Infection and Immunity, 2005; Seki, Tasaka et al., Inflammation Research, 2010; Nadigel, Prefontaine et al., Respiratory Research, 2011; Kovach and Standiford, International Immunology, 2011; Bauer, Shapiro et al., Mol Med, 2012; Deng, Yang et al., PLoS One, 2013; Freeman, Martinez et al., Respiratory Research, 2013; Dubaniewicz, A., Human Immunology, 2013). TLR as well as IL-1R family members are also involved in the pathogenesis of other inflammatory diseases such as Behcet's disease, gout, lupus erythematosus, adult Still's disease, and chronic inflammatory bowel disease such as ulcerative colitis and Crohn's disease as well as transplant rejection. Therefore, IRAK4 inhibitors are suitable for the treatment of such diseases (Nickerson, Christensen et al., The Journal of Immunology, 2010; Fig. Kobori, Yagi et al., J Gastroenterol, 2010; Shi, Mucsi et al., Immunological Reviews, 2010; Chen, Lin et al., Arthritis Res Ther, 2013; Hao, Liu et al., Curr Opin Gastroenterol, 2013; Kreisel and Gold stein, Transplant International, 2013; Li, Wang et al., Pharmacology & Therapeutics, 2013; Zhu, Jiang et al., Autoimmunity, 2013; Yap and Lai, Nephrology, 2013). IRAK4 inhibitors are also suitable for preventive and/or therapeutic use in TLR and IL-1R family-mediated diseases of endometriosis and atherosclerosis. (Akoum, Lawson et al., Human Reproduction, 2007; Allhom, Boing et al., Reproductive Biology and Endocrinology, 2008; Lawson, Bourcier et al., Journal of Reproductive Immunology, 2008; Seneviratne, Sivagurunathan et al., Clinica Chimica Acta, 2012; Khan, Kitajima et al., Journal of Obstetrics and Gynaecology Research, 2013; Santulli, Borghese et al., Human Reproduction, 2013; Sedimbi, Hagglof et al., Cell Mol Life Sci, 2013).

In addition to the above mentioned diseases, IRAK4-mediated TLR process has also been documented in the pathogenesis of ocular diseases such as retinal ischemia, keratitis, allergic conjunctivitis, dry keratoconjunctivitis, macular degeneration, and uveitis (Kaarniranta and Salminen, J Mol Med (Berl), 2009; Sun and Pearlman, Investigative Ophthalmology & Visual Science, 2009; Redfern and McDermott, Experimental Eye Research, 2010; Kezic, Taylor et al., J Leukoc Biol, 2011; Chang, McCluskey et al., Clinical & Experimental Ophthalmology, 2012; Guo, Gao et al., Immunol Cell Biol, 2012; Lee, Hattori et al., Investigative Ophthalmology & Visual Science, 2012; Qi, Zhao et al., Investigative Ophthalmology & Visual Science, 2014).

The prior art discloses many IRAK4 inhibitors (see, e.g., Annual Reports in Medicinal Chemistry (2014), 49, 117-133; Progress in Medicinal Chemistry, 2017; 56: 117-163).

IRAK4 inhibitors of indazole backbone have been extensively studied and have also been proved to be one of the effective structural types of IRAK4 inhibitors. Among them, BAY-1834845 and BAY-1830839 are in Phase I clinical research stage. The prior art has also published a series of IRAK4 inhibitors with indazole structure, such as: WO2007091107A1, WO2011153588A1, WO2013106254A1, WO2015091426A1, WO2015193846A1, WO201510466A1, WO2016083433A1, WO2016174183A1, WO2017009798A1, WO2017108744A1, WO2017157792A1, WO2017207385A1, WO2017207386A1, WO2017148902A1, WO2017207481A1, WO2018060174A1, WO2018178947A1, CN110835332A, WO2019089580A1, WO2020035019A1, WO2020048471A1. In addition, similar non-indazole IRAK4 inhibitors have been reported: WO2020035020A1, CN109890829A, CN110770229A, CN110785418A, CN111094292A, CN110835338A, WO2017207340A1, WO2018234345A1.

Although early clinical reports on IRAK4 inhibitors have been published, there is no drug for this target on the market, and only PF-6650833, BAY-1834845, BAY-1830839, R835 and CA-4948 have entered the clinical stage.

BAY-1830839 and BAY-1834845, which are in clinical stage I, are indazole IRAK4 inhibitors.

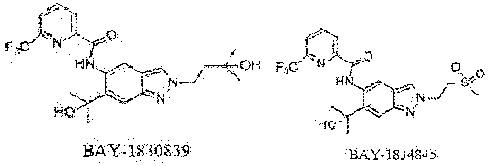

CN111362920A discloses a class of alkyl sulfoximide indazole compounds, the representative compounds are:

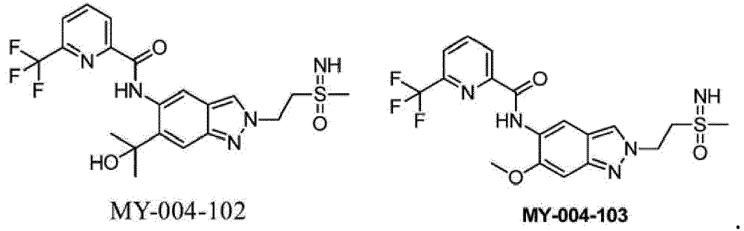

The compounds and experimental drugs disclosed in the prior art are still unsatisfactory in terms of efficacy, safety, pharmacokinetics, etc. It is still necessary to continue researching and developing new IRAK4 inhibitors to meet people's growing medical and health needs.

### SUMMARY

The technical problem to be solved by the present invention is to provide a sulfoximide substituted indazole IRAK4 kinase inhibitor with excellent IRAK4 inhibitory activity, animal safety, and pharmacokinetic parameters.

The technical solution of the present invention to solve the above-mentioned technical problem is as follows:
A sulfoximide substituted indazole compound of formula I, an isomer thereof, or a pharmaceutically acceptable salt thereof, 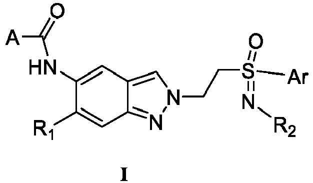 wherein
A is selected from 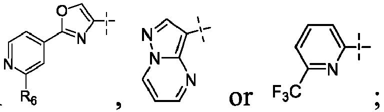
R₁ is selected from hydrogen, cyano, halogen, C₁-C₆ alkyl, C₁-C₆ alkyl hydroxyl, C₁-C₆ alkoxy or C₃-C₈ cycloalkyl;
or R₁ is morpholinyl, tetrahydropyrrolyl, morpholinyl substituted by one or more hydroxyl or C₁-C₆ alkyl, or tetrahydropyrrolyl substituted by one or more hydroxyl or C₁-C₆ alkyl;
or R1 is 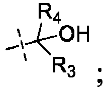
R₂ is selected from hydrogen, C₁-C₆ alkyl, or C₃-C₈ cycloalkyl; C₁-C₆ alkyl and C₃-C₈ cycloalkyl can be substituted by one or more halogen;
Ar is selected from aryl or heteroaryl, optionally substituted by one or more R₅;
R₃ and R₄ are selected from hydrogen or C₁-C₆ alkyl;
R₅ is selected from hydrogen, cyano, halogen, C₁-C₆ alkyl, C₁-C₆ alkoxy or C₃-C₈ cycloalkyl;
R₆ is selected from hydrogen, halogen or C₁-C₆ alkyl.

Preferably, Ar is selected from 5-membered to 6-membered monocyclic aromatic ring or 10-membered aromatic fused ring, which contains 0,1 or 2 heteroatoms selected from N, O, and S, and is optionally substituted by one or more R5.

Preferably, A is selected from 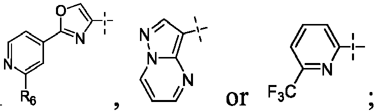
R₁ is selected from hydrogen, cyano, halogen, C₁-C₆ alkyl, C₁-C₆ alkoxy or C₃-C₈ cycloalkyl;
or R₁ is 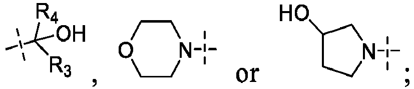
R₂ is selected from hydrogen, C₁-C₆ alkyl or C₃-C₈ cycloalkyl;
R₃ and R₄ always have the same definition, and are both selected from hydrogen or C₁-C₆ alkyl;
Ar is selected from benzene ring, pyridine ring, pyrimidine ring, quinoline ring, quinazoline ring, thiophene ring, thiazole ring or oxazole ring substituted by one or more R₅; in addition to the aromatic rings listed above, Ar can also choose other common aromatic ring structures.
R₅ is selected from hydrogen, cyano, halogen, C₁-C₆ alkyl, C₁-C₆ alkoxy or C₃-C₈ cycloalkyl;
R₆ is selected from hydrogen or C₁-C₆ alkyl.

Further preferably, A is selected from 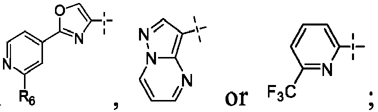
R₁ is selected from hydrogen, cyano, halogen, C₁-C₃ alkyl, C₁-C₃ alkoxy or C₃-C₈ cycloalkyl;
or R₁ is 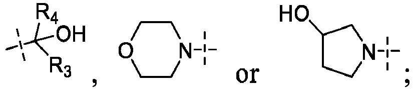
R₂ is selected from hydrogen, C₁-C₃ alkyl or C₃-C₈ cycloalkyl;
R₃ and R₄ always have the same definition, and are both selected from hydrogen or C₁-C₃ alkyl;
Ar is selected from benzene ring, pyridine ring, pyrimidine ring, quinoline ring, quinazoline ring, thiophene ring, thiazole ring or oxazole ring substituted by one, two or three R₅;
R₅ is selected from hydrogen, cyano, halogen or C₁-C₃ alkyl;
R₆ is selected from hydrogen or C₁-C₃ alkyl.

"C₁-C₃ alkyl" in the application refers to methyl, ethyl, n-propyl or isopropyl; "C₁-C₃ alkoxy" refers to methoxy, ethoxy, n-propoxy, and isopropoxy; "halogen" refers to F, Cl, Br, I; "C₃-C₈ cycloalkyl" refers to cyclopropyl, cyclobutyl, cyclopentyl, cyclohexyl, cycloheptyl or cyclooctyl.

The typical compounds of the present invention include, but are not limited to, the compounds listed in Table 1.

**Table 1**

| **Compound** | **Structural formula** | **Compound** | **Structural formula** |
|---|---|---|---|
| **I-1** | 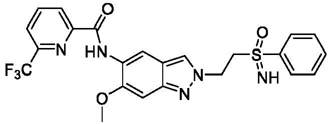 | **I-2** | 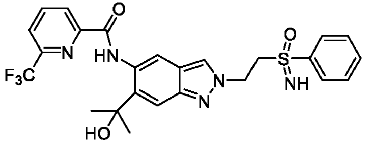 |
| **I-3** | 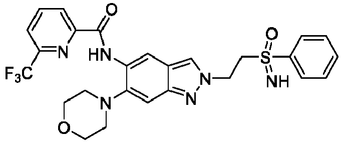 | **I-4** | 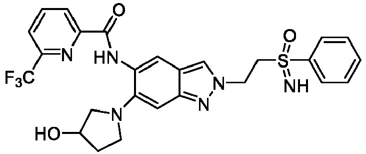 |
| **I-5** | 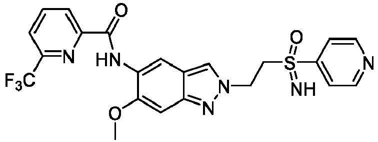 | **I-6** | 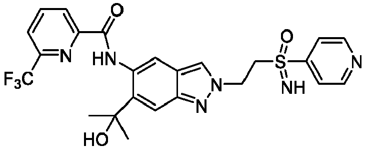 |
| **I-7** | 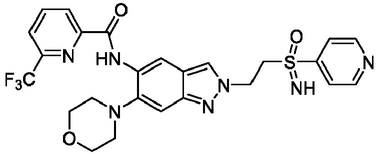 | **I-8** | 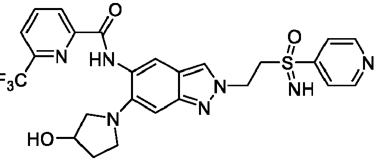 |
| **I-9** | 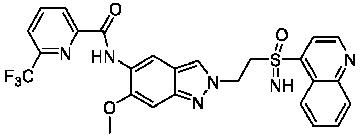 | **I-10** | 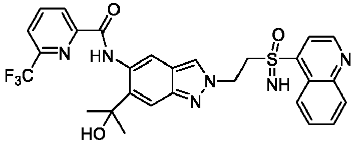 |
| **I-11** | 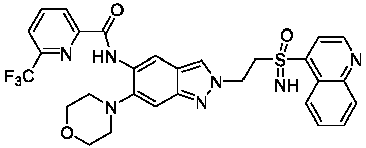 | **I-12** | 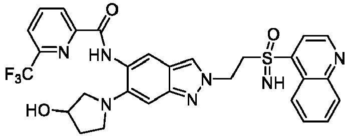 |
| **I-13** | 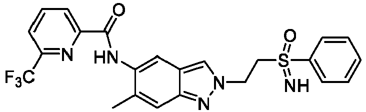 | **I-14** | 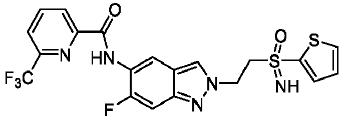 |
| **I-15** | 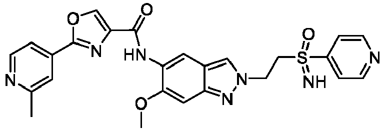 | **I-16** | 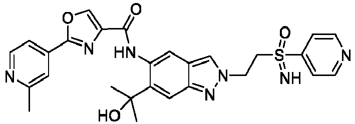 |
| **I-17** | 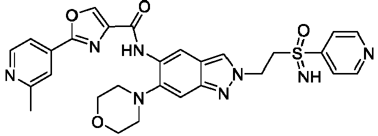 | **I-18** | 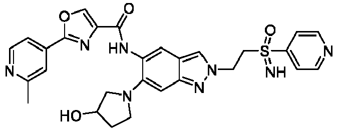 |
| **I-19** | 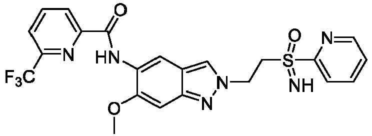 | **I-20** | 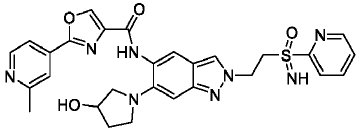 |
| **I-21** | 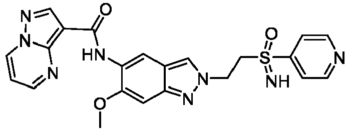 | **I-22** | 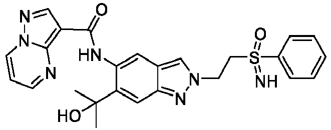 |
| **I-23** | 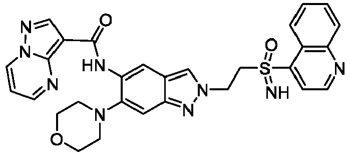 | **I-24** | 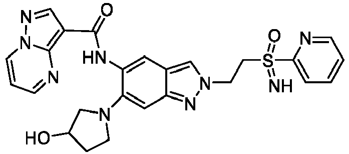 |
| **I-25** | 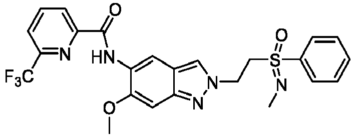 | **I-26** | 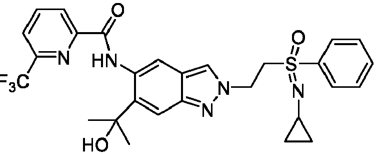 |
| **I-27** | 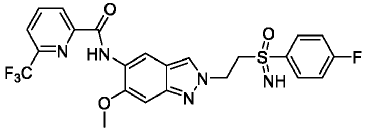 | **I-28** | 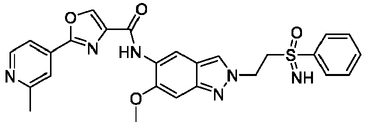 |
| **I-29** | 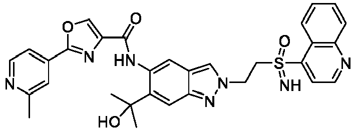 | **I-30** | 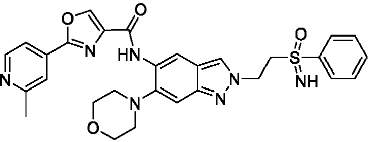 |
| **I-31** | 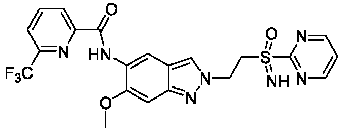 | **I-32** | 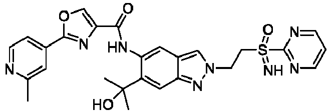 |
| **I-33** | 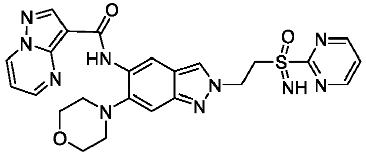 | **I-34** | 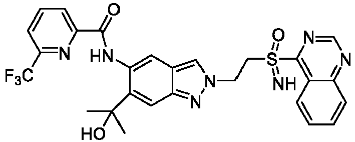 |
| **I-35** | 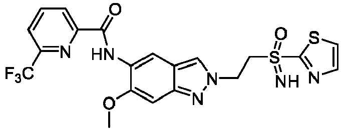 | **I-36** | 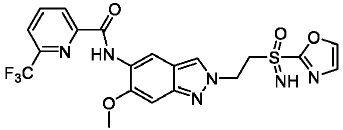 |

A second objective of the present invention is to provide a synthesis method for the above-mentioned compound: 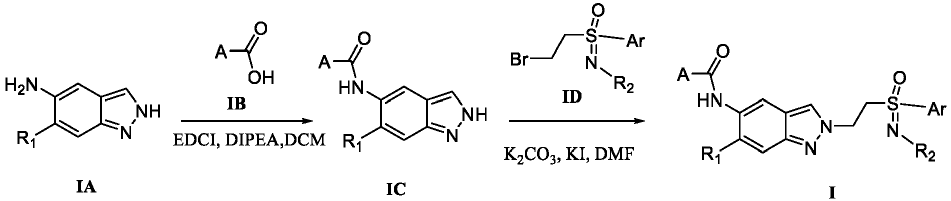
(1) performing a condensation reaction of compound IA with compound IB to produce compound IC;
(2) reacting compound IC with side chain ID to produce final product I.

The definition of each group in the reaction step is as described above.

A third aspect of the present invention provides use of the compound of formula (I), isomer thereof, or pharmaceutically acceptable salt thereof in the preparation of a drug that inhibits protein kinase.

In some embodiments, protein kinase is IRAK family kinase, especially IRAK4 kinase.

Another aspect of the present invention provides use of the compound of formula (I), isomer thereof, or pharmaceutically acceptable salt thereof in the preparation of a drug for the treatment of disease caused by protein kinase.

In some embodiments, the compound or combination of the present invention can be used to treat disease caused by IRAK family kinase, especially IRAK4 kinase: autoimmune disease; inflammatory disease; pain disease; respiratory and lung disease, lung disease particularly can be selected from lung inflammation and injury, pulmonary hypertension; gastrointestinal disease; allergic disease; infectious disease; trauma and tissue injury disease; fibrotic disease; eye disease; joint, muscle and bone disease; skin disease; kidney disease; hematopoietic system disease; liver disease; oral disease; metabolic disease; heart disease; vascular disease; neuroinflammatory disease; neurodegenerative disease; sepsis; genetic disease.

In some embodiments, the autoimmune disease and inflammatory disease are selected from systemic lupus erythematosus (SLE), lupus nephritis, arthritis, psoriasis, colitis, Crohn's disease, atopic dermatitis, liver fibrosis, myelofibrosis, thrombocythemia, polycythemia, gout, cryopyrin-associated periodic syndrome (CAPS), chronic kidney disease or acute kidney injury, chronic obstructive pulmonary disease (COPD), asthma, bronchospasm, or graft-versus-host disease.

In some embodiments, the compound or composition of the present invention can be used to treat diseases of abnormal cell proliferation caused by IRAK family kinase, especially IRAK4 kinase, especially cancer.

In some embodiments, the cancer of the present invention includes breast cancer, small cell lung cancer, non-small cell lung cancer, bronchioloalveolar carcinoma, prostate cancer, cholangiocarcinoma, bone cancer, bladder cancer, head and neck cancer, kidney cancer, liver cancer, gastrointestinal tissue cancer, esophageal cancer, ovarian cancer, pancreatic cancer, skin cancer, testicular cancer, thyroid cancer, uterine cancer, cervical and vaginal cancer, leukemia, multiple myeloma and lymphoma.

In the process of treating disease, the derivatives of the present invention can be used in the form of compositions for the treatment of related cancers and other diseases through oral administration, injection, etc. When used for oral administration, it can be prepared into conventional solid preparations such as tablets, powders or capsules. When used for injection, it can be prepared into injections.

A fourth aspect of the present invention provides a composition, comprising a therapeutically effective amount of the above novel aromatic (hetero) sulfoximide substituted indazole compound, isomer thereof, or pharmaceutically acceptable salt thereof and a pharmaceutically acceptable carrier.

The pharmaceutically acceptable salt includes, such as, salts formed with inorganic acids, salts formed with organic acids. Non-limiting examples of the salts formed with inorganic acids include, but are not limited to, salts formed with hydrochloric acid, hydrobromic acid, nitric acid, sulfuric acid, phosphoric acid, and the like. Non-limiting examples of the salts formed with organic acids include, but are not limited to, salts formed with formic acid, acetic acid, trifluoroacetic acid, fumaric acid, oxalic acid, malic acid, maleic acid, tartaric acid, citric acid, succinic acid, methanesulfonic acid, benzenesulfonic acid, p-toluenesulfonic acid, and the like.

The carrier mentioned above refers to a conventional carrier in the pharmaceutical field, which includes: diluents, excipients such as water, etc.; binders such as cellulose derivatives, gelatin, polyvinylpyrrolidone, etc.; fillers such as starch, etc.; and disintegrating agents such as calcium carbonate and sodium bicarbonate. In addition, other auxiliary agents such as flavoring agents and sweetening agents may also be added to the composition.

Various dosage forms of the composition of the present invention can be prepared by conventional methods in the medical field, wherein the content of the active ingredient is 0.1% to 99.5% by weight.

The dosage of the composition of the present invention may vary depending on the route of administration, the age and weight of the patient, the type and severity of the disease to be treated, and the like. The daily dose is 0.001-30 mg/kg body weight (oral administration) or 0.005-30 mg/kg body weight (injection).

Compared with the existing indazole IRAK4 inhibitors (such as BAY-1834845, BAY-1830839), MY-004-102 and MY-004-103 in CN111362920A, in addition to the better inhibitory activity on IRAK4, the compounds in the present invention have the following advantages: 1) the substitution of existing substituent group with sulfoximide structure has achieved unexpected effect on reducing the risk of hERG inhibition; 2) the preliminary safety of the compound in the present invention is better than that of the existing indazole IRAK4 inhibitors; 3) in vivo animal experiment date has shown that at the same dose, the pharmacokinetic parameters AUC and Cmax of the compounds of the present invention are significantly higher than those of existing indazole IRAK4 inhibitors.

Thus, the compound of the present invention has better PK properties, so it can be reasonably speculated that when the compound of the present invention is applied to clinical practice, the effective dose will be lower and the medication safety will be higher.

If the name of a compound in the present invention is in conflict with the structural formula, the structural formula shall prevail unless the structural formula is obviously wrong.

Compared with the prior art, the novel aromatic (hetero) sulfoximide substituted indazole compound, isomer thereof, or pharmaceutically acceptable salt thereof provided by the present invention shows better IRAK4 inhibitory activity and better safety. In addition, the preferred compounds of the present invention exhibit good pharmacokinetic properties and have the potential to be developed as selective IRAK4 inhibitors.

### DETAILED DESCRIPTION OF THE INVENTION

### Example 1: Synthesis of 1-1

### Synthetic route:

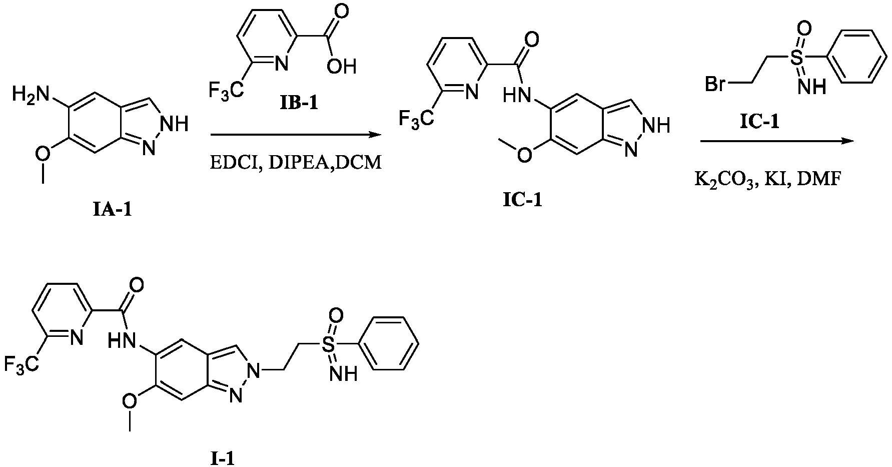

### Operation steps:

### Step 1:

To dichloromethane (DCM, 30 mL) were added compound IA-1 (1.63 g, 0.01 mol) and compound IB-1 (1.91 g, 0.01 mol), followed by N, N-diisopropylethylamine (DIPEA, 1.94 g, 0.015 mol) and 1-(3-dimethylaminopropyl)-3-ethylcarbodiimide (EDCI) (2.3 g, 0.015 mol), and the reaction system was stirred at 30°C for 12 h. The reaction mixture was extracted with water (20 mL), and the organic layer was concentrated under reduced pressure and recrystallized with absolute ethanol (10 mL) to give a yellowish solid IC-1 (2.67 g, yield 79.5%). ¹H NMR (400 MHz, DMSO-*d₆*): δ = 13.10 (br, 1H), 11.55 (br, 1H), 8.35 (d, *J =* 8.0 Hz, 1H), 8.14 (m, 1H), 7.98 (d, *J* = 8.0 Hz, 1H), 7.72 (s, 1H), 7.47 (s, 1H), 7.04 (s, 1H), 3.92 (s, 3H). LCMS: MS Calcd.: 336.3, MS Found: 337.2[M+1].

### Step 2:

Compounds **IC-1**(400 mg, 1.2 mmol), **ID-1**(327 mg, 1.32 mmol), K₂CO₃(332 mg, 2.4 mmol) and KI (17 mg, 0.1 mmol) were added to DMF (10 mL), which was heated to 100 °C under N₂ protection and stirred for 24 h. After the reaction was completed, the mixture was cooled to room temperature and concentrated under reduced pressure. The residue was passed through a silica gel column (eluent ethyl acetate/petroleum ether: 1/2), and product fraction was collected and concentrated to give product **I-1** (210 mg, yield 35%). ¹HNMR (400 MHz, DMSO-d₆): δ = 11.53 (br, 1H), 10.45 (br, 1H), 8.36 (d, *J* = 8.0 Hz, 1H), 8.12 (m, 2H), 8.02 (s, 1H), 7.71 (d, *J* = 8.0 Hz, 1H), 7.51 (m, 5H), 7.32 (s, 1H), 4.11 (t, *J* = 4.8 Hz, 2H), 3.86 (s, 3H), 3.04 (t, *J* = 4.8 Hz, 2H). LCMS: MS Calcd.: 503.5, MS Found: 504.2 [M+1].

The preparation of compounds **I-2~I-36** was carried out according to the compound **I-1** scheme in Example 1, and relevant data of the compounds are shown in Table 2.

**Table 2**

| **Compound** | **Structural formula** | **yield** | **MS** | **¹HNMR (400 MHz, DMSO-*d₆*)** |
|---|---|---|---|---|
| **I-2** | 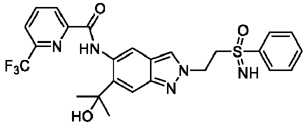 | 25% | MS Calcd.: 531.5, MS | δ = 11.53 (br, 1H), 10.43 (br, 1H), 8.37 (d, *J* = 8.0 Hz, 1H), 8.13 (m, 2H), 8.02 (s, 1H), 7.69 (m, 2H), 7.51 (m, 5H), 5.51 (br, 1H), 4.10 (t, *J* = 4.8 Hz, 2H), 3.05 (t, *J* = 4.8 Hz, 2H), 1.35 (s, 6H)_{∘} |
| | | | Found: 532.3 [M+1] | |
| **I-3** | 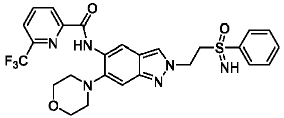 | 23% | MS Calcd.: 558.5, MS | δ = 11.51 (br, 1H), 10.41 (br, 1H), 8.37 (m, 1H), 8.13 (m, 1H), 8.03 (s, 1H), 7.92 (s, 1H), 7.69 (m, 1H), 7.51 (m, 5H), 7.03 (s, 1H), 4.07 (t, *J=* 4.8 Hz, 2H), 3.73 (m, 4H), 3.25 (m, 6H)_{∘} |
| | | | Found: 559.2 [M+1] | |
| **I-4** | 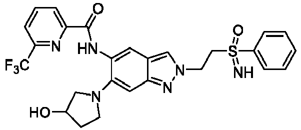 | 23% | MS Calcd.: 558.6, MS | δ = 11.51 (br, 1H), 10.43 (br, 1H), 8.37 (m, 1H), 8.14 (m, 1H), 8.03 (s, 1H), 7.93 (s, 1H), 7.69 (m, 1H), 7.50 (m, 5H), 7.03 (s, 1H), 5.37 (br, 1H), 4.06 (t, *J* = 4.8 Hz, 2H), 3.69 (m, 1H), 3.25 (m, 6H), 1.67 (m, 2H)_{∘} |
| | | | Found: 559.3[M+1] | |
| **I-5** | 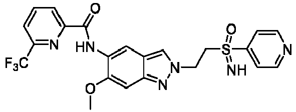 | 24% | MS Calcd.: 504.4, MS | δ = 11.53 (br, 1H), 10.46 (br, 1H), 8.75 (m, 2H), 8.37 (d, *J* = 8.0 Hz, 1H), 8.12 (m, 2H), 8.03 (s, 1H), 7.78 (m, 2H), 7.69 (m, 1H), 7.32 (s, 1H), 4.09 (t, *J* = 4.8 Hz, 2H), 3.87 (s, 3H), 3.03 (t, *J* = 4.8 Hz, 2H)_{∘} |
| | | | Found: 505.2[M+1] | |
| **I-6** | 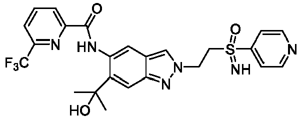 | 23% | MS Calcd.: 532.5, MS | δ = 11.51 (br, 1H), 10.45 (br, 1H), 8.75 (m, 2H), 8.37 (m, 1H), 8.13 (m, 2H), 8.03 (s, 1H), 7.78 (m, 12H), 7.69 (m, 2H), 5.53 (br, 1H), 4.08 (t, *J* = 4.8 Hz, 2H), 3.03 (t, *J* = 4.8 Hz, 2H), 1.35 (s, 6H)_{∘} |
| | | | Found: 533.2[M+1] | |
| **I-7** | 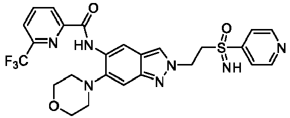 | 26% | MS Calcd.: 559.6, MS | δ = 11.53 (br, 1H), 10.45 (br, 1H), 8.76 (m, 2H), 8.37 (m, 1H), 8.13 (m, 1H), 8.03 (s, 1H), 7.92 (s, 1H), 7.78 (m, 2H), 7.03 (s, 1H), 4.06 (t, *J* = 4.8 Hz, 2H), 3.73 (m, 4H), 3.19 (m, 4H), 3.06 (t, *J* = 4.8 Hz, 2H)_{∘} |
| | | | Found: 560.3[M+1] | |
| **I-8** | 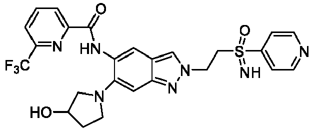 | 24% | MS Calcd.: 559.6, MS | δ = 11.52 (br, 1H), 10.46 (br, 1H), 8.75 (m, 2H), 8.37 (m, 1H), 8.13 (m, 1H), 8.03 (s, 1H), 7.92 (s, 1H), 7.75 (m, 2H), 7.68 (m, 1H), 7.02 (s, 1H), 5.36 (br, 1H), 4.09 (t, *J* = 4.8 Hz, 2H), 3.69 (m, 1H), 3.25 (m, 6H), 1.67 (m, 2H)_{∘} |
| | | | Found: 560.3[M+1] | |
| **I-9** | 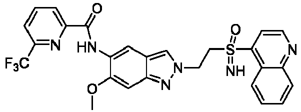 | 28% | MS Calcd.: 554.4, MS | δ = 11.51 (br, 1H), 10.45 (br, 1H), 9.12 (m, 1H), 8.37 (m, 1H), 8.12 (m, 3H), 8.01 (m, 2H), 7.83 (m, 1H), 7.69 (m, 3H), 7.32 (s, 1H), 4.09 (t, *J* = 4.8 Hz, 2H), 3.87 (s, 3H), 3.05 (t, *J* = 4.8 Hz, 2H) |
| | | | Found: 555.2[M+1] | |
| **I-10** | 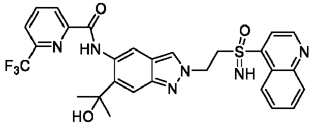 | 24% | MS Calcd.: 582.6, MS | δ = 11.51 (br, 1H), 10.45 (br, 1H), 9.13 (m, 1H), 8.37 (m, 1H), 8.13 (m, 3H), 8.03 (m, 2H), 7.83 (m, 1H), 7.67 (m, 4H), 5.53 (br, 1H), 4.07 (t, *J* = 4.8 Hz, 2H), 3.03 (t, *J* = 4.8 Hz, 2H), 1.35 (s, 6H) |
| | | | Found: 583.3[M+1] | |
| **I-11** | 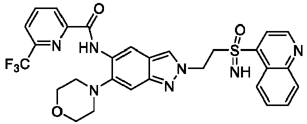 | 27% | MS Calcd.: 609.6, MS | δ = 11.54 (br, 1H), 10.44 (br, 1H), 9.12 (m, 1H), 8.37 (m, 1H), 8.13 (m, 2H), 8.01 (m, 3H), 7.83 (m, 1H), 7.68 (m, 3H), 7.03 (s, 1H), 4.08 (t, *J* = 4.8 Hz, 2H), 3.74 (m, 4H), 3.19 (m, 4H), 3.05 (t, *J* = 4.8 Hz, 2H)_{∘} |
| | | | Found: 610.3[M+1] | |
| **I-12** | 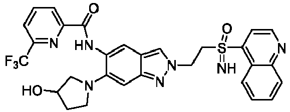 | 25% | MS Calcd.: 609.6, MS | δ = 11.53 (br, 1H), 10.44 (br, 1H), 9.12 (m, 1H), 8.37 (m, 1H), 8.14 (m, 2H), 8.01 (m, 3H), 7.82 (m, 1H), 7.68 (m, 3H), 7.03 (s, 1H), 5.37 (br, 1H), 4.09 (t, *J* = 4.8 Hz, 2H), 3.69 (m, 1H), 3.25 (m, 6H), 1.67 (m, 2H)_{∘} |
| | | | Found: 610.3[M+1] | |
| **I-13** | 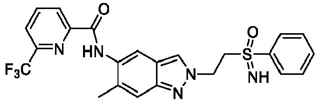 | 29% | MS Calcd.: 487.5, MS | δ = 11.53 (br, 1H), 10.45 (br, 1H), 8.37 (d, *J* = 8.0 Hz, 1H), 8.12 (m, 1H), 8.03 (m, 2H), 7.69 (m, 1H), 7.55 (m, 6H), 4.11 (t, *J* = 4.8 Hz, 2H), 3.24 (t, *J* = 4.8 Hz, 2H) , 2.15 (s, 3H)_{∘} |
| | | | Found: 488.2[M+1] | |
| **I-14** | 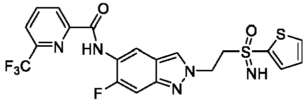 | 27% | MS Calcd.: 487.5, MS | δ = 11.52 (br, 1H), 10.45 (br, 1H), 8.37 (m, 1H), 8.10 (m, 2H), 8.03 (s, 1H), 7.69 (m, 1H), 7.48 (m, 2H), 7.05 (m, 2H), 4.09 (t, *J* = 4.8 Hz, 2H), 3.05 (t, *J* = 4.8 Hz, 2H)_{∘} |
| | | | Found: 488.2[M+1] | |
| **I-15** | 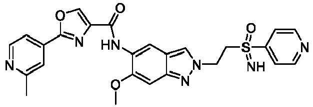 | 30% | MS Calcd.: 517.4, MS | δ = 11.53 (br, 1H), 10.46 (br, 1H), 8.88 (m, 1H), 8.75 (m, 2H), 8.24 (m, 1H), 8.10 (s, 1H), 8.03 (s, 1H), 7.78 (m, 3H), 7.69 (s, 1H), 7.32 (s, 1H), 4.09 (t, *J* = 4.8 Hz, 2H), 3.87 (s, 3H), 3.05 (t, *J* = 4.8 Hz, 2H), 2.69 (s, 3H)_{∘} |
| | | | Found: 518.2[M+1] | |
| **I-16** | 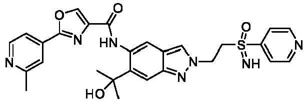 | 24% | MS Calcd.: 545.5, MS | δ = 11.51 (br, 1H), 10.45 (br, 1H), 8.88 (m, 1H), 8.75 (m, 2H), 8.24 (m, 1H), 8.11 (s, 1H), 8.03 (s, 1H), 7.78 (m, 3H), 7.69 (s, 1H), 5.53 (br, 1H), 4.07 (t, *J* = 4.8 Hz, 2H), 3.05 (t, *J* = 4.8 Hz, 2H), 2.69 (s, 3H), 1.36 (s, 6H)_{∘} |
| | | | Found: 546.2[M+1] | |
| **I-17** | 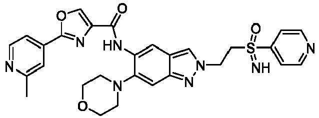 | 26% | MS Calcd.: 572.5, MS | δ = 11.52 (br, 1H), 10.45 (br, 1H), 8.88 (m, 1H), 8.76 (m, 2H), 8.25 (m, 1H), 8.02 (s, 1H), 7.92 (s, 1H), 7.78 (m, 3H), 7.68 (s, 1H) , 7.03 (s, 1H), 4.08 (t, *J* = 4.8 Hz, 2H), 3.73 (m, 4H), 3.19 (m, 4H), 3.04 (t, *J* = 4.8 Hz, 2H), 2.69 (s, 3H)_{∘} |
| | | | Found: 573.2[M+1] | |
| **I-18** | 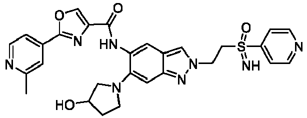 | 25% | MS Calcd.: 572.5, MS | δ = 11.51 (br, 1H), 10.45 (br, 1H), 8.87 (m, 1H), 8.75 (m, 2H), 8.25 (m, 1H), 8.03 (s, 1H), 7.92 (s, 1H), 7.79 (m, 3H), 7.68 (s, 1H), 7.03 (s, 1H), 5.36 (br, 1H), 4.09 (t, *J* = 4.8 Hz, 2H), 3.68 (m, 1H), 3.15 (m, 3H), 3.05 (m, 4H), 2.69 (s, 3H), 1.69(m, 2H)_{∘} |
| | | | Found: 573.2[M+1] | |
| **I-19** | 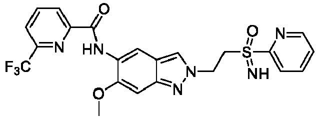 | 27% | MS Calcd.: 504.4, MS | δ = 11.52 (br, 1H), 10.46 (br, 1H), 8.64 (m, 1H), 8.37 (m, 1H), 8.12 (m, 3H), 8.03 (s, 1H), 7.78 (m, 3H), 7.69 (m, 1H), 7.32 (s, 1H), 4.09 (t, *J* = 4.8 Hz, 2H), 3.86 (s, 3H), 3.03 (t, *J* = 4.8 Hz, 2H) |
| | | | Found: 505.2[M+1] | |
| **I-20** | 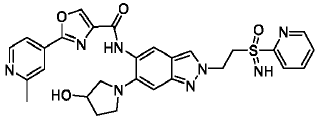 | 25% | MS Calcd.: 572.5, MS | δ = 11.53 (br, 1H), 10.45 (br, 1H), 8.88 (m, 1H), 8.64 (m, 1H), 8.25 (m, 1H), 8.03 (s, 1H), 7.92 (s, 1H), 7.83 (s, 1H), 7.71 (m, 3H), 7.03 (s, 1H), 5.35 (br, 1H), 4.09 (t, *J* = 4.8 Hz, 2H), 3.69 (m, 1H), 3.17 (m, 3H), 3.05 (m, 4H), 2.69 (s, 3H), 1.68(m, 2H)_{∘} |
| | | | Found: 573.2[M+1] | |
| **I-21** | 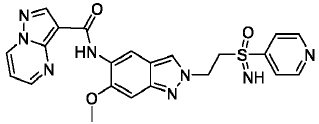 | 29% | MS Calcd.: 476.5, MS | δ = 11.52 (br, 1H), 10.45 (br, 1H), 9.25 (m, 1H), 8.81 (m, 3H), 8.38 (s, 1H), 8.10 (s, 1H), 8.03 (s, 1H), 7.78 (m, 2H), 7.30 (m, 1H), 4.08 (t, *J* = 4.8 Hz, 2H), 3.86 (s, 3H), 3.03 (t, *J* = 4.8 Hz, 2H)_{∘} |
| | | | Found: 477.3[M+1] | |
| **I-22** | 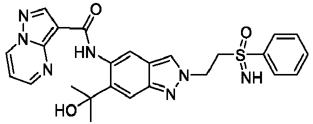 | 25% | MS Calcd.: 503.5, MS | δ = 11.53 (br, 1H), 10.43 (br, 1H), 9.25 (m, 1H), 8.83 (m, 1H), 8.38 (s, 1H), 8.13 (s, 1H), 8.03 (s, 1H), 7.70 (s, 1H), 7.50 (m, 5H), 7.25 (m, 1H), 5.52 (br, 1H), 4.10 (t, *J* = 4.8 Hz, 2H), 3.20 (t, *J* = 4.8 Hz, 2H), 1.36 (s, 6H)_{∘} |
| | | | Found: 504.3 [M+1] | |
| **I-23** | 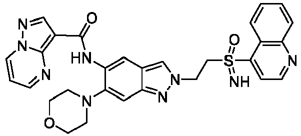 | 28% | MS Calcd.: 581.6, MS | δ = 11.53 (br, 1H), 10.44 (br, 1H), 9.25 (m, 1H), 9.12 (m, 1H), 8.37 (m, 1H), 8.13 (m, 1H), 8.01 (m, 3H), 7.83 (m, 1H), 7.68 (m, 2H), 7.28 (m, 1H), 7.03 (s, 1H), 4.09 (t, *J* = 4.8 Hz, 2H), 3.73 (m, 4H), 3.19 (m, 4H), 3.05 (t, *J* = 4.8 Hz, 2H)_{∘} |
| | | | Found: 582.2[M+1] | |
| **I-24** | 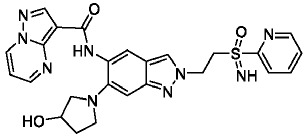 | 24% | MS Calcd.: 531.6, MS | δ = 11.54 (br, 1H), 10.46 (br, 1H), 9.25 (m, 1H), 8.83 (m, 1H), 8.64 (m, 1H), 8.37 (s, 1H), 8.05 (m, 2H), 7.92 (s, 1H), 7.71 (m, 2H), 7.28 (m, 2H), 7.03 (s, 1H), 5.36 (br, 1H), 4.09 (t, *J* = 4.8 Hz, 2H), 3.69 (m, 1H), 3.17 (m, 3H), 3.05 (m, 4H), 1.68(m, 2H)_{∘} |
| | | | Found: 532.2[M+1] | |
| **I-25** | 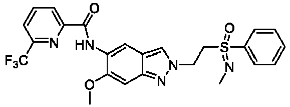 | 26% | MS Calcd.: 517.5, MS | δ = 11.52 (br, 1H), 8.37 (m, 1H), 8.12 (m, 2H), 8.03 (s, 1H), 7.69 (m, 1H), 7.52 (m, 5H), 7.32 (s, 1H), 4.10 (t, *J* = 4.8 Hz, 2H), 3.86 (s, 3H), 3.24 (t, *J* = 4.8 Hz, 2H), 0.75 (s, 3H)_{∘} |
| | | | Found: 518.3[M+1] | |
| **I-26** | 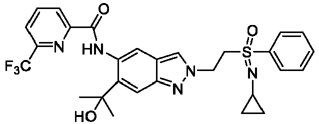 | 24% | MS Calcd.: 531.5, MS | δ = 11.53 (br, 1H), 8.36 (m, 1H), 8.12 (m, 2H), 8.13 (m, 2H), 8.03 (s, 1H), 7.70 (m, 2H), 7.53 (m, 5H), 5.51 (br, 1H), 4.10 (t, *J* = 4.8 Hz, 2H), 3.25 (t, *J* = 4.8 Hz, 2H), 1.35 (s, 6H), 0.50 (m, 5H)_{∘} |
| | | | Found: 532.3 [M+1] | |
| **I-27** | 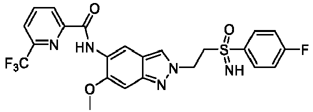 | 27% | MS Calcd.: 521.5, MS | δ = 11.52 (br, 1H), 10.46 (br, 1H), 8.36 (m, 1H), 8.12 (m, 2H), 8.03 (s, 1H), 7.70 (m, 1H), 7.42 (m, 5H), 4.10 (t, *J* = 4.8 Hz, 2H), 3.86 (s, 3H), 3.24 (t, *J* = 4.8 Hz, 2H)_{∘} |
| | | | Found: 522.3 [M+1] | |
| **I-28** | 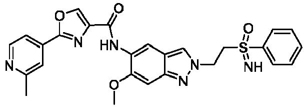 | 29% | MS Calcd.: 516.5, MS | δ = 11.53 (br, 1H), 10.46 (br, 1H), 8.88 (m, 1H), 8.24 (m, 1H), 8.12 (s, 1H), 8.02 (s, 1H), 7.83 (s, 1H), 7.69 (s, 1H), 7.52 (m, 5H), 7.32 (s, 1H), 4.09 (t, *J* = 4.8 Hz, 2H), 3.86 (s, 3H), 3.24 (t, *J* = 4.8 Hz, 2H), 2.69 (s, 3H)_{∘} |
| | | | Found: 517.3 [M+1] | |
| **I-29** | 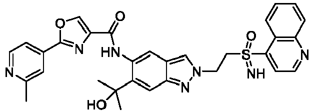 | 25% | MS Calcd.: 595.5, MS | δ = 11.51 (br, 1H), 10.45 (br, 1H), 9.12 (m, 1H), 8.88 (m, 1H), 8.25 (m, 1H), 8.14 (m, 2H), 8.02 (m, 2H), 7.83 (m, 2H), 7.68 (m, 4H), 5.53 (br, 1H), 4.08 (t, *J* = 4.8 Hz, 2H), 3.05 (t, *J* = 4.8 Hz, 2H), 2.69 (s, 3H), 1.35 (s, 6H)_{∘} |
| | | | Found: 596.3[M+1] | |
| **I-30** | 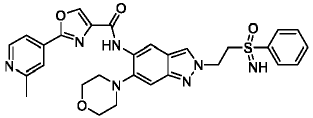 | 26% | MS Calcd.: 571.6, MS | δ = 11.53 (br, 1H), 10.44 (br, 1H), 8.87 (m, 1H), 8.24 (m, 1H), 8.03 (s, 1H), 7.92 (s, 1H), 7.83 (s, 1H), 7.69 (s, 1H), 7.52 (m, 5H), 7.03 (s, 1H), 4.09 (t, *J* = 4.8 Hz, 2H), 3.73 (m, 4H), 3.19 (m, 6H) , 2.69 (s, 3H)_{∘} |
| | | | Found: 572.2 [M+1] | |
| **I-31** | 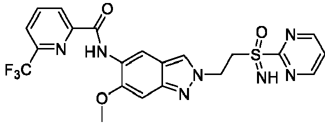 | 26% | MS Calcd.: 505.5, MS | δ = 11.51 (br, 1H), 10.46 (br, 1H), 9.14 (m, 2H), 8.37 (m, 1H), 8.10 (m, 2H), 8.03 (m, 2H), 7.69 (m, 1H), 7.32 (s, 1H), 4.09 (t, *J* = 4.8 Hz, 2H), 3.87 (s, 3H), 3.06 (t, *J* = 4.8 Hz, 2H)_{∘} |
| | | | Found: 506.2[M+1] | |
| **I-32** | 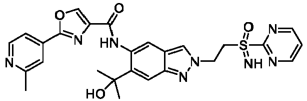 | 23% | MS Calcd.: 546.5, MS | δ = 11.52 (br, 1H), 10.45 (br, 1H), 9.15 (m, 2H), 8.88 (m, 1H), 8.24 (m, 1H), 8.11 (s, 1H), 8.03 (m, 2H), 7.81 (s, 1H), 7.70 (m, 2H), 5.53 (br, 1H), 4.07 (t, *J* = 4.8 Hz, 2H), 3.06 (t, *J* = 4.8 Hz, 2H), 2.69 (s, 3H), 1.36 (s, 6H)_{∘} |
| | | | Found: 547.2[M+1] | |
| **I-33** | 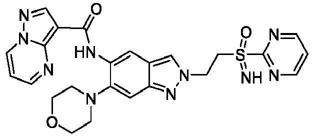 | 25% | MS Calcd.: 532.5, MS | δ = 11.53 (br, 1H), 10.44 (br, 1H), 9.25 (m, 1H), 9.14 (m, 2H), 8.83 (m, 1H), 8.38 (s, 1H), 7.95 (m, 3H), 7.28 (m, 1H), 7.03 (s, 1H), 4.09 (t, *J* = 4.8 Hz, 2H), 3.73 (m, 4H), 3.19 (m, 4H), 3.05 (t, *J* = 4.8 Hz, 2H)_{∘} |
| | | | Found: 533.2[M+1] | |
| **I-34** | 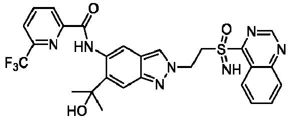 | 25% | MS Calcd.: 583.5, MS | δ = 11.51 (br, 1H), 10.44 (br, 1H), 9.43 (s, 1H), 8.37 (m, 1H), 8.13 (m, 3H), 8.03 (s, 1H), 7.83 (m, 2H), 7.65 (m, 3H), 5.53 (br, 1H), 4.07 (t, *J* = 4.8 Hz, 2H), 3.03 (t, *J* = 4.8 Hz, 2H), 1.35 (s, 6H) |
| | | | Found: 584.3[M+1] | |
| **I-35** | 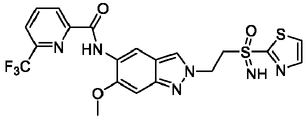 | 25% | MS Calcd.: 510.5, MS | δ = 11.52 (br, 1H), 10.45 (br, 1H), 8.37 (m, 1H), 8.12 (m, 2H), 8.03 (s, 1H), 7.69 (m, 2H), 7.54 (m, 1H), 7.32 (s, 1H), 4.08 (t, *J* = 4.8 Hz, 2H), 3.86 (s,3H), 3.03 (t, *J* = 4.8 Hz, 2H)_{∘} |
| | | | Found: 511.2[M+1] | |
| **I-36** | 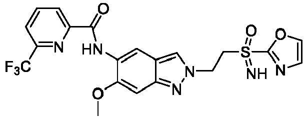 | 27% | MS Calcd.: 494.4, MS | δ = 11.51 (br, 1H), 10.45 (br, 1H), 8.37 (m, 1H), 8.13 (m, 2H), 8.03 (s, 1H), 7.68 (m, 2H), 7.54 (m, 1H), 7.32 (s, 1H), 4.08 (t, *J* = 4.8 Hz, 2H), 3.85 (s, 3H), 3.03 (t, *J* = 4.8 Hz, 2H)_{∘} |
| | | | Found: 495.2[M+1] | |

### Biological Test

### Test Example 1: IRAK4 kinase inhibitory activity assay

The inhibitory activity (IC50) of the compound on IRAK4 kinase under Km ATP was detected by mobility shift assay (MSA). Ten drug concentration gradients were set (initial concentration 1µM, 3-fold dilution, 2 duplicate wells per concentration). IRAK4 kinase was added to the kinase buffer solution, which was transfered to a test plate, and then FAM-labeled peptide and ATP (37 µM) were added. After incubation at 28°C for a period of time, 10 µL of termination buffer was added to terminate the reaction. The conversion rate data was read with Caliper, and then the conversion rate was converted into inhibition rate data. According to the inhibition rate data of each concentration, the IC50 of half inhibitory concentration was calculated by Logit method (Table 3).

**Table 3 Experimental test results of the inhibitory activity of the compounds on IRAK4 kinase**

| **Compound** | **IRAK4 IC₅₀ (nM)** | **Compound** | **IRAK4 IC₅₀ (nM)** | **Compound** | **IRAK4 IC₅₀ (nM)** |
|---|---|---|---|---|---|
| BAY-1834845 | 15.2 | BAY-1830839 | 8.7 | MY-004-102 | 152 |
| MY-004-103 | 137 | **I-1** | 0.3 | **I-2** | 0.5 |
| **I-3** | 0.6 | **I-4** | 0.3 | **I-5** | 0.4 |
| **I-6** | 0.2 | **I-7** | 0.5 | **I-8** | 0.6 |
| **I-9** | 0.2 | **I-10** | 1.1 | **I-11** | 0.5 |
| **I-12** | 0.3 | **I-13** | 0.4 | **I-14** | 0.6 |
| **I-15** | 0.7 | **I-16** | 0.5 | **I-17** | 0.5 |
| **I-18** | 0.4 | **I-19** | 0.8 | **I-20** | 0.6 |
| **I-21** | 0.5 | **I-22** | 0.3 | **I-23** | 0.4 |
| **I-24** | 0.8 | **I-25** | 0.5 | **I-26** | 0.3 |
| **1-27** | 0.7 | **I-28** | 0.7 | **I-29** | 0.6 |
| **I-30** | 0.6 | **I-31** | 0.6 | **I-32** | 0.5 |
| **I-33** | 0.4 | **I-34** | 0.5 | **I-35** | 0.7 |
| **I-36** | 0.5 | | | | |

| | | | | | |
|---|---|---|---|---|---|
| Note: The results of the controls and the compounds of the present invention were measured under the same experimental conditions. | | | | | |

Conclusion: The inhibitory activity of the compound of the present invention on IRAK4 kinase is significantly better than that of the comparative compounds BAY-1834845, BAY-1830839, MY-004-102, and MY-004-103.

### Test Example 2: The ability of the compounds to inhibit TNF-α secretion in THP-1 cells

With the help of this test, it can be suitable for testing the ability of the compounds to inhibit TNF-α (tumor necrosis factor α) secretion in THP-1 cell (Tohoku Hospital Pediatrics-1). TNF-α is a key cytokine involved in the inflammatory process of the listed autoimmune disease, such as rheumatoid arthritis, psoriatic arthritis, ankylosing spondylitis, psoriasis, Crohn's disease, ulcerative colitis, etc. In this test, TNF-α secretion was triggered by incubation with bacterial lipopolysaccharides (LPS).

150 µL RPMI-1640 culture medium solution containing 10000 THP-1 cells was added to each well of the 96-well plate, and then 25 µL test compound solution containing 8 times the final concentration (starting from 10 µM, 3 times dilution, 9 concentrations, each concentration containing 4% DMSO RPMI-1640 culture medium) was added, which was mixed and incubated at 37°C for 30 minutes. 25 µL RPMI-1640 culture medium solution containing LPS (final LPS concentration was 1 µg/mL, final DMSO concentration was 0.5%) was added to each test well, mixed, and incubated at 37°C for 4.5 hours. The 96-well plate was rotated at 2000 rpm for 5 minutes, and then 50 µL of supernatant was taken. The content of TNF-α in the supernatant was determined by human ELISA kit, and IC₅₀ value of the compound was calculated by XL-Fit (table 4).

**Table 4 The activity of the compound of the present invention in inhibiting LPS-stimulated TNF-α secretion in THP-1 cells**

| **Compound** | **IC₅₀ (nM)** | **Compound** | **IC₅₀ (nM)** | **Compound** | **IC₅₀ (nM)** |
|---|---|---|---|---|---|
| BAY-1834845 | 19.3 | BAY-1830839 | 16.2 | MY-004-102 | 169 |
| MY-004-103 | 125 | **I-1** | 0.5 | **I-2** | 0.3 |
| **I-3** | 0.6 | **I-4** | 1.3 | **I-5** | 0.7 |
| **I-6** | 0.6 | **I-7** | 1.2 | **I-8** | 0.9 |
| **I-9** | 1.0 | **I-10** | 1.0 | **I-11** | 0.8 |
| **I-12** | 0.6 | **I-13** | 1.1 | **I-14** | 0.7 |
| **I-15** | 0.9 | **I-16** | 0.9 | **I-17** | 1.2 |
| **I-18** | 1.4 | **I-19** | 0.9 | **I-20** | 1.1 |
| **I-21** | 0.9 | **I-22** | 0.8 | **I-23** | 1.1 |
| **I-24** | 0.8 | **I-25** | 1.2 | **I-26** | 1.5 |
| **I-27** | 1.3 | **I-28** | 0.7 | **I-29** | 0.9 |
| **I-30** | 0.9 | **I-31** | 1.3 | **I-32** | 1.0 |
| **I-33** | 1.4 | **I-34** | 0.5 | **I-35** | 0.7 |
| **I-36** | 1.5 | | | | |

| | | | | | |
|---|---|---|---|---|---|
| Note: The results of the controls and the compounds of the present invention were measured under the same experimental conditions. | | | | | |

Conclusion: The compound of the present invention can effectively inhibit LPS-stimulated TNF-α secretion in THP-1 cells, and the inhibitory effect is significantly better than the comparison compounds BAY-1834845, BAY-1830839, MY-004-102 and MY-004-103.

### Test Example 3: Pharmacokinetic test of the compounds of the present invention

SD rats were used as the test animals. After the rats were intragastrically administered BAY-1834845, BAY-1830839, MY-004-102, MY-004-103 and the compounds of the preferred examples of the present invention, the drug concentration in the plasma at different times was determined through LC/MS/MS method to study the pharmacokinetic characteristics of the compounds of the present invention in rats.
Source of SD rats: Shanghai Slack Laboratory Animal Co., Ltd.
Administration: Single intragastric administration
Dosage and concentration: 10 mg/kg; 2 mg/mL
Prescription of preparations: 0.5% methylcellulose
Sampling points: 5 min, 15 min, 30 min, 1 h, 2 h, 4 h, 8 h, and 24 h.

### Standard curve and preparation and processing of quality control samples:

Stock solution was diluted with 50% acetonitrile-water to obtain standard working solutions of 0.04, 0.10, 0.20, 0.40, 1.00, 2.00, 4.00 µg/mL, and quality control working solutions of 0.10, 1.00, 3.00 µg/mL. 2.50 µL of standard curve working solution and quality control working solution were added to 47.5 µL of blank rat plasma, respectively, to obtain standard curve solutions having analyte concentrations of 2.00, 5.00, 10.00, 20.00, 50.00, 100.00, 200.00 ng/mL and quality control samples having concentrations of 5.00, 50.00, and 150.00 ng/mL. 200 µL of acetonitrile (containing 5 ng/mL of the internal standard loratadine) was added. After the vortex oscillation for 3 minutes, the solutions were centrifuged at 15000 rpm at 4°C for 15 minutes. 100 µL of the supernatant was taken for LC-MS/MS analysis. WinNonlin^{®} 8.0 was used to calculate the experimental results.

The pharmacokinetic parameters of the preferred compounds of the present invention are shown in Table 5.

**Table 5: Pharmacokinetic parameters of preferred compounds**

| Compound | Pharmacokinetic experiment (25 mg/kg) | | | |
|---|---|---|---|---|
| | Time to peak | Blood concentration | Curve area | Half life |
| | Tₘₐₓ (h) | Cₘₐₓ (ng/mL) | AUCₗₐₛₜ (h*ng/mL) | t_{1/2} (h) |
| BAY-1834845 | 1.5 | 201 | 659 | 1.4 |
| BAY-1830839 | 1.5 | 223 | 621 | 1.3 |
| MY-004-102 | 1.3 | 235 | 700 | 1.4 |
| MY-004-103 | 1.4 | 245 | 734 | 1.4 |
| I-1 | 6.2 | 1605 | 8330 | 5.0 |
| 1-2 | 6.5 | 1585 | 8100 | 5.5 |
| 1-4 | 6.0 | 1500 | 8530 | 4.7 |
| 1-5 | 6.2 | 1400 | 8280 | 5.1 |
| 1-7 | 5.0 | 1505 | 8630 | 5.0 |
| 1-9 | 5.5 | 1423 | 8522 | 4.8 |
| 1-10 | 6.3 | 1508 | 8998 | 4.7 |
| 1-14 | 5.8 | 1520 | 7983 | 5.5 |
| 1-15 | 5.9 | 1379 | 8230 | 6.0 |
| 1-16 | 6.0 | 1360 | 9230 | 6.3 |
| 1-17 | 5.8 | 1430 | 8200 | 5.4 |
| 1-20 | 4.9 | 1398 | 7500 | 4.5 |
| 1-21 | 5.8 | 1520 | 9405 | 5.2 |
| 1-24 | 6.2 | 1415 | 8900 | 5.5 |
| 1-26 | 5.7 | 1479 | 8321 | 5.6 |
| 1-29 | 5.9 | 1369 | 8129 | 5.8 |
| 1-31 | 4.8 | 1297 | 7980 | 4.5 |
| 1-34 | 5.5 | 1630 | 8000 | 5.3 |
| 1-36 | 5.6 | 1492 | 8490 | 5.4 |

Conclusion: The compounds of the examples of the present invention exhibited good pharmacokinetic properties and had significant pharmacokinetic advantages over BAY-1834845, BAY-1830839, MY-004-102 and MY-004-103.

### Test Example 4: Acute toxicity test of the compounds of the present invention

Compounds of the present invention (1-1, I-2, I-5, I-7, 1-10, 1-16, I-21, I-24, I-26, I-29, 1-31, I-34) and MY-004-102, BAY-1830839 (positive control drug) were chosen for acute toxicity experiments.

### (1) Experimental protocol

(1) After oral administration of MY-004-102, BAY-1830839 and compounds of the present invention such as I-1 to ICR mice, the toxicity symptoms and death of mice were observed. The acute toxicity of MY-004-102, BAY-1830839 and compounds of the present invention such as I-1 was compared.
② Solvent preparation: An appropriate amount of methylcellulose (MC) was weighed and diluted with ultrapure water to a certain volume to obtain 5% MC (w/v).
③ Administration preparation: The predetermined test samples were weighed and added with 5% MC solution to obtain suspensions having the concentration of 12.5, 37.5, 75.00 and 100.00 mg/mL.
④ Administration route: The test samples and the controls (0.5% MC) were administrated orally.
⑤ Administration frequency: single administration, fasting overnight before administration.

### Observation of general symptoms:

On the day of administration, observations were made approximately 0.5, 1, 2, 4, and 6 hours after the first administration. During day 2 to day 6 of the observation period, observations were made twice a day, once in the morning and once in the afternoon.

Observations included but not limited to: general conditions, behavioral activities, gait and posture, eyes, mouth, nose, gastrointestinal tract, skin hair and urogenital tract.

### (2) Statistical analysis

Weight data were expressed as mean ± standard deviation. Levene's test and one-way analysis of variance were used for comparison between groups. If there was a difference, Dunnet t test was then used.

### (3) Experimental results

compounds of the present invention such as I-1 and MY-004-102, BAY-1830839 (positive control drug) were chosen for acute toxicity experiments as described above. The experimental results are shown in Table 6.

In the MTD test, the animal's tolerance to drugs was observed. The maximum tolerated dose refers to the dose administered when the animal was dying.

**Table 6: Acute toxicity test results of single oral administration of compounds such as I-1 and MY-004-102, BAY-1830839**

| Test compound | MTD (mg/kg) |
|---|---|
| MY-004-102 | 750 |
| BAY-1830839 | 750 |
| I-1 | >2000 |
| 1-2 | >2000 |
| 1-5 | >2000 |
| 1-7 | >2000 |
| 1-10 | >2000 |
| 1-16 | >2000 |
| 1-21 | >2000 |
| 1-24 | >2000 |
| 1-26 | >2000 |
| 1-29 | >2000 |
| 1-31 | >2000 |
| 1-34 | >2000 |

| | |
|---|---|
| Note: MTD represents maximum tolerated dose. | |

Results: The MTD (maximum tolerated dose) of the selected compounds such as I-1 of the present invention were all greater than 2000 mg/kg, and the acute toxicity was far lower than that of MY-004-102, BAY-1830839.

### Test Example 5: The effect of the compound of the present invention on the hERG potassium channel

The rapid activation of delayed rectifier outward potassium current (I_{Kr}) in humans is mainly mediated by hERG ion channel and participates in human myocardial cell repolarization. Drug blockade of this current will lead to the appearance of QT interval prolongation syndrome in clinical practice, which can easily induce acute arrhythmia and even sudden death. This study used manual patch clamp techniques to test the effect of compounds such as I-1 on hERG potassium currents in stable cell lines transfected with hERG potassium channel, thereby to determine whether compounds such as I-1 have inhibitory effect on hERG ion channel.

The final concentrations of the test compounds are 5 µM, 50 µM, and 500 µM. The final concentration of DMSO in extracellular fluid is 0.3%. The inhibitory effect of the test compound on bERG current is shown in Table 7.

**Table 7: The effect of the compounds such as I-1 and MY-004-102, BAY-1830839 on hERG potassium channel**

| Test compound | Inhibition rate (%) | | |
|---|---|---|---|
| | 5 µM | 50 µM | 500 µM |
| MY-004-102 | 8.7% | 56.7% | 99.8% |
| BAY-1830839 | 7.2% | 51.2% | 102.4% |
| 1-1 | -1.0% | 2.3% | 15.6% |
| 1-2 | -0.5% | 0.7% | 12.3% |
| 1-5 | 0.0% | -1.0% | 13.4% |
| 1-10 | 0.1 | 0.9% | 20.3% |
| 1-21 | 2.3% | 2.1% | 29.7% |
| 1-26 | 1.2% | 0.7% | 11.0% |
| 1-29 | -3.9% | 1.2% | 13.9% |
| 1-31 | 1.7% | -0.2% | 6.7% |
| 1-34 | -1.6% | 1.0% | 11.5% |

Under the conditions of this experiment, the average inhibition rate of the compounds such as I-1 of the present invention on hERG potassium channel current at 500 µM is less than 50% (N=3), so the IC₅₀ value of the inhibitory effect of compounds such as 1-1 of the present invention on hERG current is greater than 500 µM. Comparing the average inhibition rate of MY-004-102 and BAY-1830839 on hERG potassium channel current at 50 µM is over 50% (N=3), it is speculated that the IC₅₀ value of the inhibitory effect of MY-004-102 and BAY-1830839 on hERG current is around 50 µM.

The preferred examples of the present invention are described above, but they are not intended to limit the present invention. Any modification, equivalent replacement, improvement, etc. made within the spirit and principle of the present invention should be within the protection scope of the present invention.

## Claims

1. A sulfoximide substituted indazole compound of formula I, an isomer thereof, or a pharmaceutically acceptable salt thereof, 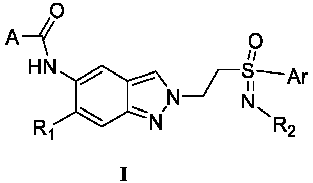 wherein
A is selected from 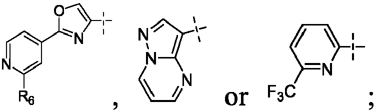
R₁ is selected from hydrogen, cyano, halogen, C₁-C₆ alkyl, C₁-C₆ alkyl hydroxyl, C₁-C₆ alkoxy or C₃-C₈ cycloalkyl;
or R₁ is morpholinyl, tetrahydropyrrolyl, morpholinyl substituted by one or more hydroxyl or C₁-C₆ alkyl, or tetrahydropyrrolyl substituted by one or more hydroxyl or C₁-C₆ alkyl;
or R1 is 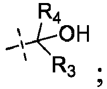
R₂ is selected from hydrogen, C₁-C₆ alkyl, or C₃-C₈ cycloalkyl; C₁-C₆ alkyl and C₃-C₈ cycloalkyl can be substituted by one or more halogen;
Ar is selected from aryl or heteroaryl, optionally substituted by one or more R₅;
R₃ and R₄ are selected from hydrogen or C₁-C₆ alkyl;
R₅ is selected from hydrogen, cyano, halogen, C₁-C₆ alkyl, C₁-C₆ alkoxy or C₃-C₈ cycloalkyl;
R₆ is selected from hydrogen, halogen or C₁-C₆ alkyl.

2. The sulfoximide substituted indazole compound, isomer thereof, or pharmaceutically acceptable salt thereof according to claim 1, wherein
A is selected from 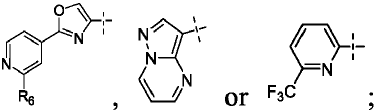
R₁ is selected from hydrogen, cyano, halogen, C₁-C₆ alkyl, C₁-C₆ alkoxy or C₃-C₈ cycloalkyl;
or R₁ is 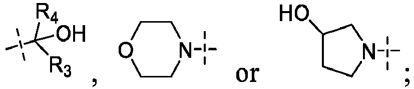
R₂ is selected from hydrogen, C₁-C₆ alkyl or C₃-C₈ cycloalkyl;
R₃ and R₄ are selected from hydrogen or C₁-C₆ alkyl;
Ar is selected from benzene ring, pyridine ring, pyrimidine ring, quinoline ring, quinazoline ring, thiophene ring, thiazole ring or oxazole ring substituted by one or more R₅;
R₅ is selected from hydrogen, cyano, halogen, C₁-C₆ alkyl, C₁-C₆ alkoxy or C₃-C₈ cycloalkyl;
R₆ is selected from hydrogen or C₁-C₆ alkyl.

3. The sulfoximide substituted indazole compound, isomer thereof, or pharmaceutically acceptable salt thereof according to claim 1, wherein
A is selected from 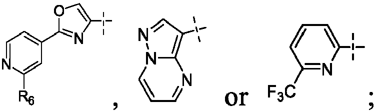
R₁ is selected from hydrogen, cyano, halogen, C₁-C₆ alkyl, C₁-C₃ alkoxy or C₃-C₈ cycloalkyl;
or R₁ is 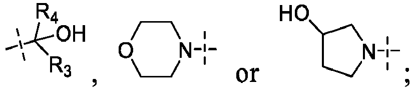
R₂ is selected from hydrogen, C₁-C₃ alkyl or C₃-C₈ cycloalkyl;
R₃ and R₄ always have the same definition, and are both selected from hydrogen or C₁-C₃ alkyl;
Ar is selected from benzene ring, pyridine ring, pyrimidine ring, quinoline ring, quinazoline ring, thiophene ring, thiazole ring or oxazole ring substituted by one, two or three R₅;
R₅ is selected from hydrogen, cyano, halogen or C₁-C₃ alkyl;
R₆ is selected from hydrogen or C₁-C₃ alkyl.

4. The sulfoximide substituted indazole compound, isomer thereof, or pharmaceutically acceptable salt thereof according to claim 1, wherein the structure of the sulfoximide substituted indazole compound is shown in the table below:
**Table 1**
| **Compound** | **Structural formula** | **Compound** | **Structural formula** |
|---|---|---|---|
| **I-1** | 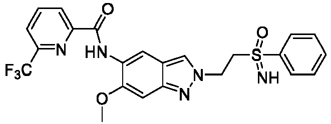 | **I-2** | 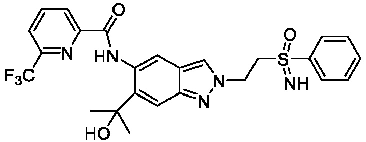 |
| **I-3** | 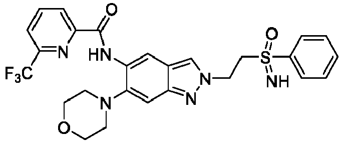 | **I-4** | 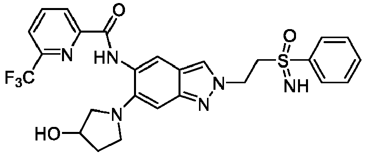 |
| **I-5** | 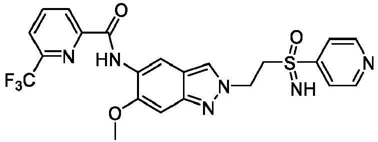 | **I-6** | 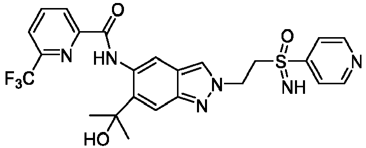 |
| **I-7** | 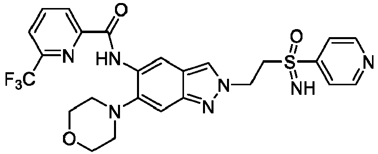 | **I-8** | 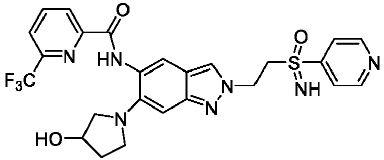 |
| **I-9** | 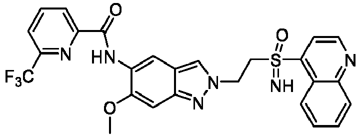 | **I-10** | 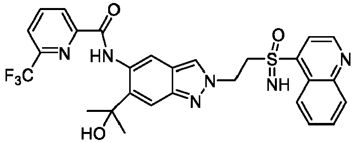 |
| **I-11** | 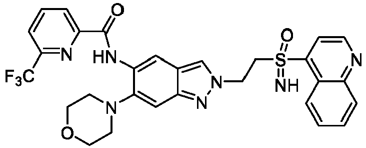 | **I-12** | 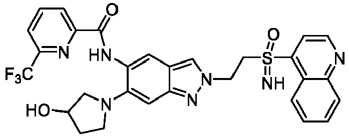 |
| **I-13** | 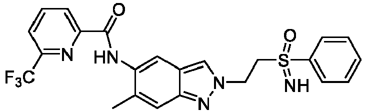 | **I-14** | 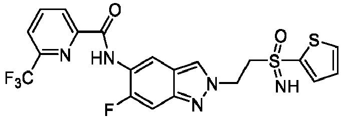 |
| **I-15** | 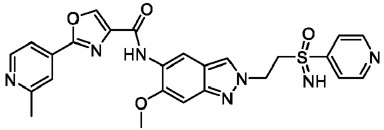 | **I-16** | 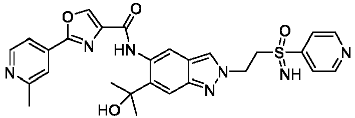 |
| **I-17** | 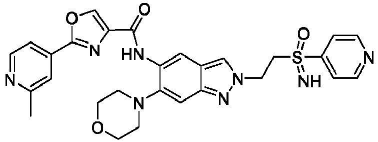 | **I-18** | 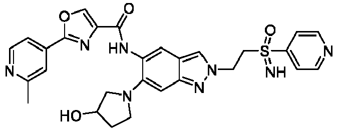 |
| **I-19** | 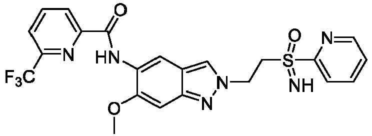 | **I-20** | 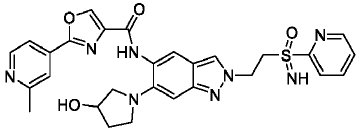 |
| **I-21** | 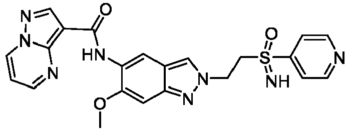 | **I-22** | 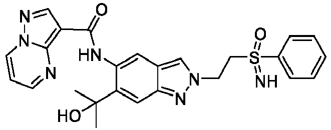 |
| **I-23** | 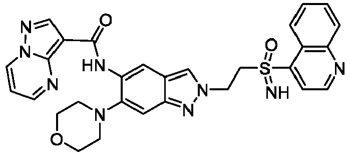 | **I-24** | 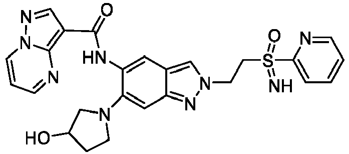 |
| **I-25** | 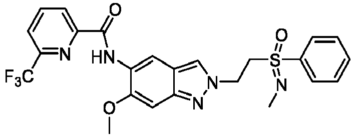 | **I-26** | 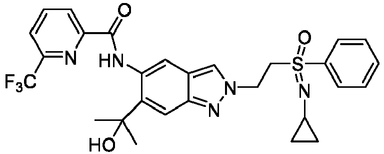 |
| **1-27** | 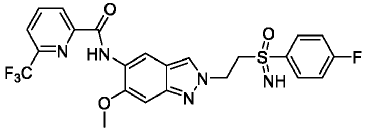 | **I-28** | 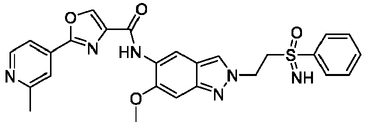 |
| **I-29** | 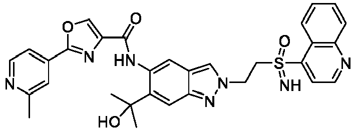 | **I-30** | 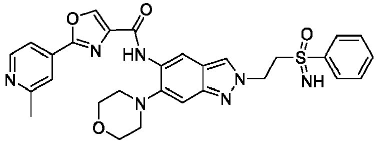 |
| **I-31** | 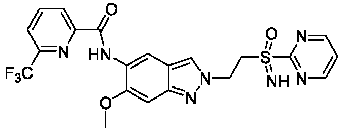 | **I-32** | 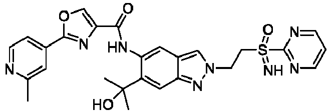 |
| **I-33** | 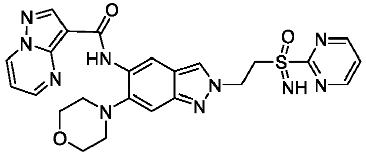 | **I-34** | 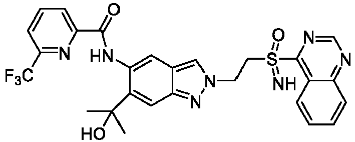 |
| **I-35** | 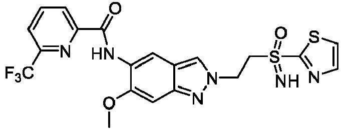 | **I-36** | 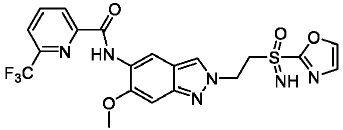 |

5. A method for preparing the sulfoximide substituted indazole compound according to anyone of claims 1-4, comprising the steps of:
(1) performing a condensation reaction of compound IA with compound IB to produce compound IC;
(2) reacting compound IC with side chain ID to produce final product I; and the synthetic scheme of the method is as follows:

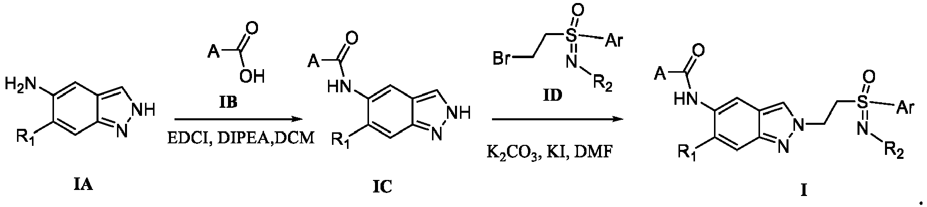

6. Use of the sulfoximide substituted indazole compound, isomer thereof, or pharmaceutically acceptable salt thereof according to anyone of claims 1-4 in the preparation of a drug for the prevention or treatment of IRAK4-related disease.

7. The use according to claim 6, wherein the disease is selected from autoimmune disease, inflammatory disease, pain disease, respiratory and lung disease, gastrointestinal disease, allergic disease, infectious disease, trauma and tissue injury disease, fibrotic disease, eye disease, joint, muscle and bone disease, skin disease, kidney disease, hematopoietic system disease, liver disease, oral disease, metabolic disease, heart disease, vascular disease, neuroinflammatory disease, neurodegenerative disease, sepsis, genetic disease, and cancer.

8. The use according to claim 7, wherein the autoimmune disease and inflammatory disease are selected from systemic lupus erythematosus, lupus nephritis, arthritis, psoriasis, colitis, Crohn's disease, atopic dermatitis, liver fibrosis, myelofibrosis, thrombocythemia, polycythemia, gout, cryopyrin-associated periodic syndrome, chronic kidney disease or acute kidney injury, chronic obstructive pulmonary disease, asthma, bronchospasm, or graft-versus-host disease.

9. The use according to claim 7, wherein the cancer is selected from breast cancer, small cell lung cancer, non-small cell lung cancer, bronchioloalveolar carcinoma, prostate cancer, cholangiocarcinoma, bone cancer, bladder cancer, head and neck cancer, kidney cancer, liver cancer, gastrointestinal tissue cancer, esophageal cancer, ovarian cancer, pancreatic cancer, skin cancer, testicular cancer, thyroid cancer, uterine cancer, cervical and vaginal cancer, leukemia, multiple myeloma or lymphoma.

10. A composition, comprising a therapeutically effective amount of the sulfoximide substituted indazole compound, isomer thereof, or pharmaceutically acceptable salt thereof according to anyone of claims 1-4, and a pharmaceutically acceptable carrier.
